# EUROPEAN PATENT SPECIFICATION

(11) **EP 3 886 788 B1**
(45) Date of publication and mention of the grant of the patent: **22.01.2025**
(21) Application number: 19816470.9
(22) Date of filing: 06.11.2019
(51) Int. Cl.: A61K 8/06, A61K 8/39, A61K 8/37, A61Q 19/00

(54) **COMPOSITION COMPRISING TWO POLYGLYCERYL FATTY ACID ESTERS**
ZUSAMMENSETZUNG, DIE ZWEI POLYGLYCERYLFETTSÄUREESTER ENTHÄLT
COMPOSITION COMPRENANT DEUX ESTERS D'ACIDES GRAS POLYGLYCÉRYLIQUES

(30) Priority: 27.11.2018 JP 2018221079
(43) Date of publication of application: 06.10.2021
(73) Proprietor: L'OREAL, 75008 Paris (FR); Niimi, Rui, Takatsu-ku Kawasaki-shi Kanagawa 213-0012 (JP); Gilles, Quentin, Kawasaki-shi, Kanagawa 2130012 (JP)
(72) Inventor: NIIMI, Rui, Kawasaki-shi Kanagawa 2130012 (JP); GILLES, Quentin, Kawasaki-shi Kanagawa 2130012 (JP)
(74) Representative: Casalonga
(86) International application number: PCT/JP2019/044447
(87) International publication number: WO 2020/110716

(56) References cited:
- WO-A1-2014/098266
- WO-A1-2016/091939
- WO-A1-2019/244908

## Description

### TECHNICAL FIELD

The present invention is as defined in the appended claims and ' relates to a composition, preferably a cosmetic or dermatological composition, which comprises at least two different types of polyglyceryl fatty acid esters.

### BACKGROUND ART

Oil-in-water (O/W) or water-in-oil (W/O) emulsions are well known in the field of cosmetics and dermatology, in particular for the preparation of cosmetic products, such as milks, creams, tonics, serums or toilet waters.

In particular, a fine emulsion such as an O/W nano- or micro-emulsion is particularly interesting in cosmetic products due to its transparent or slightly translucent aspect.

On the other hand, compositions including a polyglyceryl fatty acid ester have been known in the fields of cosmetics and dermatology. Polyglyceryl fatty acid esters can function as surfactants, and therefore, they may be used to prepare, typically, emulsions such as oil-in-water (O/W) or water-in-oil (W/O) emulsions. Polyglyceryl fatty acid esters are preferable for environmental reasons, such as low environmental load, as compared to polyoxyethylene-based surfactants.

### DISCLOSURE OF INVENTION

However, compositions which include a polyglyceryl fatty acid ester tend to be sticky and often provide a sticky feeling to the touch.

An objective of the present invention is to provide a composition in the form of a nano- or micro-emulsion which can be less sticky, even if the composition includes a polyglyceryl fatty acid ester.

The above objective of the present invention can be achieved by a composition comprising:
(a) at least one oil;
(b) at least one first polyglyceryl fatty acid ester having an HLB value of 13.0 or more, preferably 13.5 or more, and more preferably 14.0 or more;
(c) at least one second polyglyceryl fatty acid ester having an HLB value of 10.0 or less, preferably 9.0 or less, and more preferably 8.0 or less; and
(d) water,
wherein
the composition has a turbidity of 150 NTU or less, preferably 130 NTU or less, and more preferably 110 NTU or less.

The average HLB of the (b) first polyglyceryl fatty acid ester(s) and the (c) second polyglyceryl fatty acid ester(s) in the composition according to the present invention may range from 11.5 to 14.0, preferably from 12.0 to 13.5, and more preferably from 12.5 to 13.0.

The (a) oil may be selected from polar oils.

The amount of the (a) oil(s) in the composition according to the present invention may range from 0.01% to 20% by weight, preferably from 0.05% to 15% by weight, and more preferably from 0.1 % to 10% by weight, relative to the total weight of the composition.

The (b) first polyglyceryl fatty acid ester may comprise 2 to 4 glycerol units, preferably 3 or 4 glycerol units, and more preferably 4 glycerol units.

The fatty acid moiety of the (b) first polyglyceryl fatty acid ester may comprise 12 or fewer carbon atoms, preferably 11 or fewer carbon atoms, and more preferably 10 or fewer carbon atoms.

The weight ratio of the amount of the (b) first polyglyceryl fatty acid ester(s)/the amount of the (a) oil(s) in the composition according to the present invention may range from 0.6 to 1.3, preferably from 0.7 to 1.2, and more preferably from 0.8 to 1.1.

The (c) second polyglyceryl fatty acid ester may comprise 2 to 4 glycerol units, preferably 2 or 3 glycerol units, and more preferably 2 glycerol units.

The fatty acid moiety of the (c) second polyglyceryl fatty acid ester may comprise 14 or more carbon atoms, preferably 16 or more carbon atoms, and more preferably 18 or more carbon atoms.

The weight ratio of the amount of the (c) second polyglyceryl fatty acid ester(s)/the amount of the (a) oil(s) in the composition according to the present invention may range from 0.1 to 0.9, preferably from 0.2 to 0.7, and more preferably from 0.3 to 0.5.

The weight ratio of
(the total amounts of the (b) first polyglyceryl fatty acid ester(s) and the (c) second polyglyceryl fatty acid ester(s))/the amount of the (a) oil(s)

in the composition according to the present invention may be 1.6 or less, preferably 1.5 or less, and more preferably 1.4 or less.

The composition according to the present invention may be free from a polyglyceryl fatty acid ester comprising 5 or more glycerol units.

The composition according to the present invention may be in the form of an O/W emulsion. The particle size of the (a) oil in the emulsion may be 35 run or less, preferably 30 nm or less, and more preferably 25 nm or less.

The present invention also relates to a cosmetic process for treating a keratin substance, comprising the step of applying the composition according to the present invention to the keratin substance.

### BEST MODE FOR CARRYING OUT THE INVENTION

After diligent research, the inventors have discovered that it is possible to provide a composition in the form of a nano- or micro-emulsion which can be less sticky, and therefore, it can provide a reduced sticky feeling after application, even if the composition includes a polyglyceryl fatty acid ester, by using a combination of two different types of polyglyceryl fatty acid esters.

The composition according to the present invention may be characterized by a combination of
at least one first polyglyceryl fatty acid ester having a higher HLB value; and
at least one second polyglyceryl fatty acid ester having a lower HLB value.

The higher HLB value of the first polyglyceryl fatty acid ester may belong to a higher HLB numerical range,
the lower HLB value of the second polyglyceryl fatty acid ester may belong to a lower HLB numerical range,
   and
the higher HLB numerical range and the lower HLB numerical range do not overlap each other.

Thus, one aspect of the present invention is a composition comprising:
(a) at least one oil;
(b) at least one first polyglyceryl fatty acid ester having an HLB value of 13.0 or more, preferably 13.5 or more, and more preferably 14.0 or more;
(c) at least one second polyglyceryl fatty acid ester having an HLB value of 10.0 or less, preferably 9.0 or less, and more preferably 8.0 or less; and
(d) water,
wherein
the composition has a turbidity of 150 NTU or less, preferably 130 NTU or less, and more preferably 110 NTU or less.

The composition according to the present invention may be transparent or translucent because it has a turbidity of 150 NTU or less, preferably 130 NTU or less, and more preferably 110 NTU or less.

The composition according to the present invention can be less sticky, and therefore, it can provide a reduced sticky feeling to the touch. Therefore, the composition according to the present invention can provide an excellent feeling during use, in particular, feeling of the skin after application of the composition.

The term "sticky" here means a property which provides a tacky feeling to the skin.

The composition according to the present invention may be prepared without a large amount of energy such as required by a homogenizer. Thus, the composition according to the present invention may be prepared by using a small amount of energy such as gently stirring the ingredients of the composition. Therefore, the composition according to the present invention is environmentally friendly in view of the preparation approach thereof.

Hereinafter, the composition according to the present invention will be explained in a more detailed manner.

### [Oil]

The composition according to the present invention comprises (a) at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The (a) oil may be a non-polar oil such as a hydrocarbon oil, a silicone oil, or the like; a polar oil such as a plant or animal oil and an ester oil or an ether oil; or a mixture thereof.

The (a) oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids.

It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopahnitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula: with D": 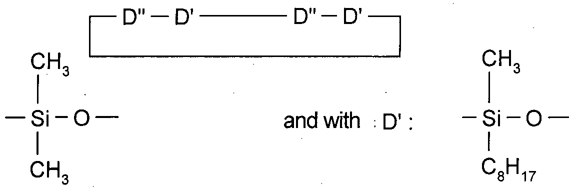 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics. The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Coming, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 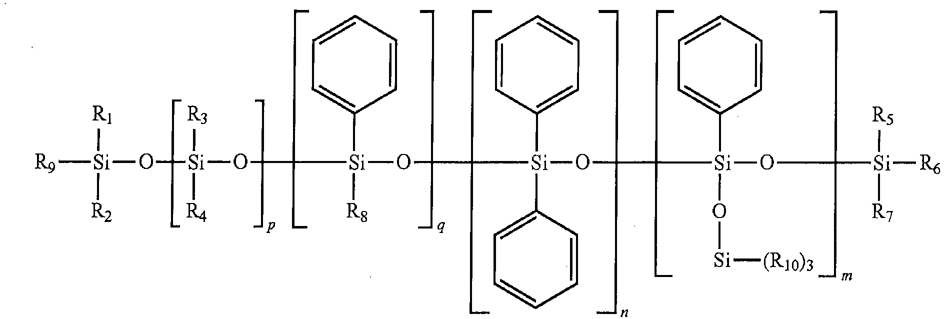 in which
Ri to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Coming 556 Cosmetic Grade Fluid from Dow Corning;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (Ri to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

The hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from cetyl alcohol, octyldodecanol, hexyldecanol, and mixtures thereof.

It is also preferable that the (a) oil be chosen from oils with a molecular weight below 600 g/mol.

Preferably, the (a) oil has a low molecular weight such as below 600 g/mol, chosen among ester oils with a short hydrocarbon chain or chains (C₁-C₁₂) (e.g., isopropyl lauroyl sarcosinate, isopropyl myristate, isopropyl palmitate, isononyl isononanoate, and ethyl hexyl palmitate), silicone oils (e.g., volatile silicones such as cyclohexasiloxane), hydrocarbon oils (e.g., isododecane, isohexadecane, and squalane), branched and/or unsaturated fatty alcohol (C₁₂-C₃₀) type oils such as octyldodecanol and oleyl alcohol, and ether oils such as dicaprylyl ether.

It is preferable that the (a) oil be chosen from polar oils, and more preferably from ester oils.

The amount of the (a) oil(s) in the composition according to the present invention may be 0.01% by weight or more, preferably 0.05% by weight or more, and more preferably 0.1% by weight or more, relative to the total weight of the composition.

The amount of the (a) oil(s) in the composition according to the present invention may be 20% by weight or less, preferably 15% by weight or less, and more preferably 10% by weight or less, relative to the total weight of the composition.

The amount of the (a) oil(s) in the composition according to the present invention may be from 0.01% to 20% by weight, preferably from 0.05% to 15% by weight, and more preferably from 0.1% to 10% by weight, relative to the total weight of the composition.

### [First Polyglyceryl Fatty Acid Ester]

The composition according to the present invention comprises (b) at least one first polyglyceryl fatty acid ester having an HLB value of 13.0 or more, preferably 13.5 or more, and more preferably 14.0 or more. A single type of(b) first polyglyceryl fatty acid ester may be used, but two or more different types of(b) first polyglyceryl fatty acid ester may be used in combination.

The (b) first polyglyceryl fatty acid ester can function as a surfactant, in particular a nonionic surfactant.

The (b) first polyglyceryl fatty acid may have an HLB value of 13.0 to 17.0, preferably 13.5 to 16.0, and more preferably 14.0 to 15.0.

The term HLB ("hydrophilic-lipophilic balance") is well known to those skilled in the art, and reflects the ratio between the hydrophilic part and the lipophilic part in the molecule.

If two or more (b) first polyglyceryl fatty acid esters are used, the HLB value is determined by the weight average of the HLB values of all the (b) first polyglyceryl fatty acid esters.

The (b) first polyglyceryl fatty acid ester may be chosen from mono, di, tri and more esters of saturated or unsaturated fatty acid(s).

It is preferable that the (b) first polyglyceryl fatty acid ester comprises 2 to 4 glycerol units, preferably 3 or 4 glycerol units, and more preferably 4 glycerol units.

The fatty acid for the fatty acid moiety or the fatty acid moiety of the (b) first polyglyceryl fatty acid ester may comprise 12 or fewer carbon atoms, preferably 11 or fewer carbon atoms, and more preferably 10 or fewer carbon atoms. The fatty acid for the fatty acid moiety or the fatty acid moiety of the (b) first polyglyceryl fatty acid ester may comprise 4 or more carbon atoms, preferably 6 or more carbon atoms, and more preferably 8 or more carbon atoms. The fatty acid for the fatty acid moiety or the fatty acid moiety of the (b) first polyglyceryl fatty acid ester may have carbon atoms of from 4 to 12, preferably from 6 to 11, and more preferably from 8 to 10 carbon atoms.

The fatty acid for the fatty acid moiety of the (b) first polyglyceryl fatty acid ester may be saturated or unsaturated, and may be selected from caprylic acid, capric acid, and lauric acid.

The (b) first polyglyceryl fatty acid ester(s) may be selected from the group consisting of PG3 caprate (HLB: about 14), PG4 caprylate (HLB: 14), PG4 laurate (HLB: about 14), PG4 caprate (HLB: 14), PG5 myristate (HLB: 15.4), PG5 stearate (HLB: 15), PG6 caprylate (HLB: 14.6), PG6 caprate (HLB: 13.1), PG6 laurate (HLB: 14.1), PG10 laurate (HLB: 15.2), PG10 myristate (HLB: 14.9), PG10 stearate (HLB: 14.1), PG10 isostearate (HLB: 13.7), PG10 oleate (HLB: 13.0), PG10 cocoate (HLB: 16), and mixtures thereof.

It may be preferable that the (b) first polyglyceryl fatty acid ester(s) be selected from the group consisting of PG3 caprate (HLB: about 14), PG4 caprylate (HLB: 14), PG4 laurate (HLB: about 14), PG4 caprate (HLB: 14), and mixtures thereof.

The amount of the (b) first polyglyceryl fatty acid ester(s) in the composition according to the present invention may be 0.01% by weight or more, preferably 0.05% by weight or more, and more preferably 0.1% by weight or more, relative to the total weight of the composition.

On the other hand, the amount of the (b) first polyglyceryl fatty acid ester(s) in the composition according to the present invention may be 15% by weight or less, preferably 10% by weight or less, and more preferably 5% by weight or less, relative to the total weight of the composition.

The amount of the (b) first polyglyceryl fatty acid ester(s) in the composition according to the present invention may range from 0.01% to 15% by weight, preferably from 0.05% to 10% by weight, and more preferably from 0.1% to 5% by weight, relative to the total weight of the composition.

The weight ratio of the amount of the (b) first polyglyceryl fatty acid ester(s)/the amount of the (a) oil(s) in the composition according to the present invention may range from 0.6 to 1.3, preferably from 0.7 to 1.2, and more preferably from 0.8 to 1.1.

### [Second Polyglyceryl Fatty Acid Ester]

The composition according to the present invention comprises (c) at least one second polyglyceryl fatty acid ester having an HLB value of 10.0 or less, preferably 9.0 or less, and more preferably 8.0 or less. A single type of (c) second polyglyceryl fatty acid ester may be used, but two or more different types of(c) second polyglyceryl fatty acid ester may be used in combination.

The (c) second polyglyceryl fatty acid ester can function as a surfactant, in particular a nonionic surfactant.

The (c) second polyglyceryl fatty acid may have an HLB value of 5.0 to 10.0, preferably 6.0 to 9.0, and more preferably 7.0 to 8.0.

If two or more (c) second polyglyceryl fatty acid esters are used, the HLB value is determined by the weight average of the HLB values of all the (c) second polyglyceryl fatty acid esters.

The (c) second polyglyceryl fatty acid ester may be chosen from mono, di, tri and more esters of saturated or unsaturated fatty acid(s).

It is preferable that the (c) second polyglyceryl fatty acid ester comprises 2 to 4 glycerol units, preferably 2 or 3 glycerol units, and more preferably 2 glycerol units.

The fatty acid for the fatty acid moiety or the fatty acid moiety of the (c) second polyglyceryl fatty acid ester may comprise 14 or more carbon atoms, preferably 16 or more carbon atoms, and more preferably 18 or more carbon atoms. The fatty acid for the fatty acid moiety or the fatty acid moiety of the (c) second polyglyceryl fatty acid ester may comprise 30 or fewer carbon atoms, preferably 24 or fewer carbon atoms, and more preferably 20 or fewer carbon atoms. The fatty acid for the fatty acid moiety or the fatty acid moiety of the (c) second polyglyceryl fatty acid ester may have from 14 to 30, preferably from 16 to 24, and more preferably from 18 to 20 carbon atoms.

The fatty acid for the fatty acid moiety of the (c) second polyglyceryl fatty acid ester may be saturated or unsaturated, and may be selected from myristic acid, stearic acid, isostearic acid, and oleic acid.

The (c) second polyglyceryl fatty acid ester(s) may be selected from the group consisting of PG2 stearate (HLB: 5.0), PG2 distearate (HLB: 4), PG2 isostearate (HLB: 8), PG2 diisostearate (HLB: 3.2), PG2 triisostearate (HLB: 3), PG2 sesquiisostearate (HLB: about 4), PG2 oleate (HLB: 8), PG2 sesquioleate (HLB: 5.3), PG3 distearate (HLB: 5), PG3 diisostearate (HLB: 5), PG3 dicocoate (HLB: 7), PG5 hexastearate (HLB: 4.0), PG5 trioleate (HLB: 7.0), PG10 pentaoleate (HLB: 6.4), PG2 sesquicaprylate (HLB: about 8), PG2 caprate (HLB: 9.5), PG2 laurate (HLB: 8.5), PG2 myristate (HLB: 10), PG2 isopalmitate (HLB: 9), PG4 oleate (HLB: 10), PG4 stearate (HLB: 9), PG4 isostearate (HLB: 8.2), PG6 distearate (HLB: 8), PG10 distearate (HLB: about 9), PG10 tristearate (HLB: 8), PG10 diisostearate (HLB: 10), PG10 triisostearate (HLB: 8), PG10 tricocoate (HLB: 9), and mixtures thereof.

It may be preferable that the (c) second polyglyceryl fatty acid be selected from the group consisting of PG2 stearate (HLB: 5.0), PG2 distearate (HLB: 4), PG2 isostearate (HLB: 8), PG2 diisostearate (HLB: 3.2), PG2 triisostearate (HLB: 3), PG2 sesquiisostearate (HLB: about 4), PG2 oleate (HLB: 8), PG2 sesquioleate (HLB: 5.3), PG3 distearate (HLB: 5), PG3 diisostearate (HLB: 5), PG3 dicocoate (HLB: 7), PG2 sesquicaprylate (HLB: about 8), PG2 caprate (HLB: 9.5), PG2 laurate (HLB: 8.5), PG2 myristate (HLB: 10), PG2 isopalmitate (HLB: 9), PG4 oleate (HLB: 10), PG4 stearate (HLB: 9), PG4 isostearate (HLB: 8.2), and mixtures thereof.

The amount of the (c) second polyglyceryl fatty acid ester(s) in the composition according to the present invention may be 0.01% by weight or more, preferably 0.05% by weight or more, and more preferably 0.1% by weight or more, relative to the total weight of the composition.

On the other hand, the amount of the (c) second polyglyceryl fatty acid ester(s) in the composition according to the present invention may be 10% by weight or less, preferably 5% by weight or less, and more preferably 1% by weight or less, relative to the total weight of the composition.

The amount of the (c) second polyglyceryl fatty acid ester(s) in the composition according to the present invention may range from 0.01% to 10% by weight, preferably from 0.05% to 5% by weight, more preferably from 0.1% to 1% by weight, relative to the total weight of the composition.

The weight ratio of the amount of the (c) second polyglyceryl fatty acid ester(s)/the amount of the (a) oil(s) in the composition according to the present invention may range from 0.1 to 0.9, preferably from 0.2 to 0.7, and more preferably from 0.3 to 0.5.

### [Amount of Polyglyceryl Fatty Acid Ester(s)]

According to the present invention, the weight ratio of (the total amounts of the (b) first polyglyceryl fatty acid ester(s) and the (c) second polyglyceryl fatty acid ester(s))/the amount of the (a) oil(s) may be 1.6 or less, preferably 1.5 or less, and more preferably 1.4 or less.

Conventionally, the weight ratio of the amount(s) of glyceryl fatty acid ester(s)/the triglyceride oil(s) is much higher, such as 6.0.

Therefore, the composition according to the present invention can reduce or limit the total amounts of the polyglyceryl fatty acid esters.

Since the present invention can reduce the total amounts of the polyglyceryl fatty acid esters, the composition according to the present invention can provide no sticky feeling or can provide a further reduced sticky feeling to the touch.

It may be preferable that the weight ratio of (the total amounts of the (b) first polyglyceryl fatty acid ester(s) and the (c) second polyglyceryl fatty acid ester(s))/the amount of the (a) oil(s) be more than 0.1, preferably more than 0.5, and more preferably more than 1.0.

### [Average HLB of Polyglyceryl Fatty Acid Ester(s)]

The average HLB of the (b) first polyglyceryl fatty acid ester(s) and the (c) second polyglyceryl fatty acid ester(s) in the composition according to the present invention may be 11.5 or more, preferably 12.0 or more, and more preferably 12.5 or more.

The average HLB of the (b) first polyglyceryl fatty acid ester(s) and the (c) second polyglyceryl fatty acid ester(s) in the composition according to the present invention may be 14.0 or less, preferably 13.5 or less, and more preferably 13.0 or less.

Thus, in one embodiment of the present invention, the average HLB of the (b) first polyglyceryl fatty acid ester(s) and the (c) second polyglyceryl fatty acid ester(s) in the composition according to the present invention may range from 11.5 to 14.0, preferably from 12.0 to 13.5, and more preferably from 12.5 to 13.0.

### [Water]

The composition according to the present invention comprises (d) water.

The amount of the (d) water in the composition according to the present invention may be 60% by weight or more, preferably 70% by weight or more, and more preferably 80% by weight or more, relative to the total weight of the composition.

On the other hand, the amount of the (d) water in the composition according to the present invention may be 95% by weight or less, preferably 90% by weight or less, and more preferably 85% by weight or less, relative to the total weight of the composition.

The amount of(d) water in the composition according to the present invention may range from 60% to 95% by weight, preferably from 70% to 90% by weight, more preferably from 80% to 85% by weight, relative to the total weight of the composition.

### [Polyol]

The composition according to the present invention may further comprise at least one polyol. A single type of polyol may be used, but two or more different types of polyol may be used in combination.

The term "polyol" here means an alcohol having two or more hydroxy groups, and does not encompass a saccharide or a derivative thereof. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acyl group or a carbonyl group.

The polyol may be a C₂-C ₁₂ polyol, preferably a C₂-C₉ polyol, comprising at least 2 hydroxy groups, and preferably 2 to 5 hydroxy groups.

The polyol may be a natural or synthetic polyol. The polyol may have a linear, branched or cyclic molecular structure.

The polyol may be selected from glycerins and derivatives thereof, and glycols and derivatives thereof. The polyol may be selected from the group consisting of glycerin, diglycerin, polyglycerin, ethyleneglycol, diethyleneglycol, propyleneglycol, dipropyleneglycol, butyleneglycol, pentyleneglycol, hexyleneglycol, 1,3-propanediol, 1,5-pentanediol, polyethyleneglycol (5 to 50 ethyleneoxide groups), and sugars such as sorbitol.

The amount of the polyol(s) in the composition according to the present invention may be 0.0 1 % by weight or more, preferably 0.05% by weight or more, and more preferably 0.1% by weight or more, relative to the total weight of the composition.

On the other hand, the amount of the polyol(s) in the composition according to the present invention may be 25% by weight or less, preferably 20% by weight or less, and more preferably 15% by weight or less, relative to the total weight of the composition.

Thus, the polyol(s) may be present in the composition according to the present invention in an amount ranging from 0.01% to 25% by weight, and preferably from 0.05% to 20% by weight, such as from 0.1% to 15% by weight, relative to the total weight of the composition.

### [Other Ingredients]

The composition according to the present invention may contain one or more monoalcohols which are in the form of a liquid at room temperature (25°C), such as for example linear or branched monoalcohols comprising from 1 to 6 carbon atoms, such as ethanol, propanol, butanol, isopropanol, isobutanol, pentanol, and hexanol.

The amount of the monoalcohol(s) in the composition according to the present invention may be 0.01% by weight or more, preferably 0.1% by weight or more, and more preferably 1% by weight or more, relative to the total weight of the composition.

On the other hand, the amount of the monoalcohol(s) in the composition according to the present invention may be 15% by weight or less, preferably 10% by weight or less, and more preferably 5% by weight or less, relative to the total weight of the composition.

Thus, the amount of the monoalcohol(s) in the composition according to the present invention may range from 0.01% to 15% by weight, preferably from 0.1% to 10% by weight, and more preferably from 1% to 5% by weight, relative to the total weight of the composition.

The composition according to the present invention may also include various adjuvants conventionally used in cosmetic and dermatological compositions, such as thickeners, anionic, non-ionic, cationic, and amphoteric or zwitterionic polymers, anionic, non-ionic, cationic, and amphoteric surfactants, antioxidants, coloring agents, chelating agents, sequestering agents, fragrances, dispersing agents, conditioning agents, film-forming agents, preservatives, co-preservatives, and mixtures thereof, except for the ingredients as explained above.

In one embodiment, the composition according to the present invention is free from a polyglyceryl fatty acid ester comprising 5 or more glycerol units such as PG5 laurate. The term "free from" here means that the composition according to the present invention may contain a limited amount of a polyglyceryl fatty acid ester comprising 5 or more glycerol units. However, it is preferable that the amount of the polyglyceryl fatty acid ester comprising 5 or more glycerol units be limited such that it is less than 1% by weight, more preferably less than 0.1% by weight, and even more preferably less than 0.01% by weight, relative to the total weight of the composition. It is most preferable that the composition according to the present invention comprise no polyglyceryl fatty acid ester comprising 5 or more glycerol units.

In another embodiment, the composition according to the present invention is free from a polyoxyethylene-based nonionic surfactant. The term "free from" here means that the composition according to the present invention may contain a limited amount of a polyoxyethylene-based nonionic surfactant. However, it is preferable that the amount of the polyoxyethylene-based nonionic surfactant be limited such that it is less than 1% by weight, more preferably less than 0.1% by weight, and even more preferably less than 0.01% by weight, relative to the total weight of the composition. It is most preferable that the composition according to the present invention comprises no polyoxyethylene-based nonionic surfactant.

### (Preparation)

The composition according to the present invention can be prepared by mixing the essential ingredient(s) as explained above, and optional ingredient(s), if necessary, as explained above.

The method and means to mix the above essential and optional ingredients are not limited. Any conventional method and means can be used to mix the above essential and optional ingredients to prepare the composition according to the present invention.

The composition according to the present invention may be prepared without a large amount of energy such as required by a homogenizer. Thus, the composition according to the present invention may be prepared by using a small amount of energy such as gently stirring the ingredients of the composition. Therefore, the composition according to the present invention is environmentally friendly in view of the preparation approach thereof.

### [Form]

The composition according to the present invention is in the form of a nano- or micro-emulsion.

The "micro-emulsion" may be defined in two ways, namely, in a broad sense and in a narrow sense. That is to say, there is the one case ("micro-emulsion in the narrow sense") in which the micro-emulsion refers to a thermodynamically stable isotropic single liquid phase containing a ternary system having three ingredients of an oily component, an aqueous component and a surfactant, and there is the second case ("micro-emulsion in the broad sense") in which among thermodynamically unstable typical emulsion systems the micro-emulsion additionally includes those such emulsions presenting transparent or translucent appearances due to their smaller particle sizes (Satoshi Tomomasa, et al., Oil Chemistry, Vol. 37, No. 11 (1988), pp. 48-53). The "micro-emulsion" as used herein refers to a "micro-emulsion in the narrow sense", i.e., a thermodynamically stable isotropic single liquid phase.

The micro-emulsion refers to either one state of an O/W (oil-in-water) type microemulsion in which oil is solubilized by micelles, a W/O (water-in-oil) type microemulsion in which water is solubilized by reverse micelles, or a bicontinuous microemulsion in which the number of associations of surfactant molecules are rendered infinite so that both the aqueous phase and oil phase have a continuous structure.

The micro-emulsion may have a dispersed phase with a particle size of 100 nm or less, preferably 50 nm or less, and more preferably 20 nm or less, measured by laser granulometry.

The "nano-emulsion" here means an emulsion characterized by a dispersed phase with a size of less than 350 nm, the dispersed phase being stabilized by a crown of the (b) to (d) nonionic surfactants that may optionally form a liquid crystal phase of lamellar type, at the dispersed phase/continuous phase interface. In the absence of specific opacifiers, the transparency of the nano-emulsions arises from the small size of the dispersed phase, this small size being obtained by virtue of the use of mechanical energy.

Nanoemulsions can be distinguished from microemulsions by their structure. Specifically, micro-emulsions are thermodynamically stable dispersions formed from, for example, micelles which are formed by the ingredients (b) and (c) and swollen with the ingredient (a). Furthermore, microemulsions do not require substantial mechanical energy in order to be prepared.

The nano-emulsion may have a dispersed phase with a particle size of 300 nm or less, preferably 200 nm or less, and more preferably 100 nm or less, measured by laser granulometry.

It is preferable that the composition according to the present invention be in the form of an O/W emulsion which comprises oil phases dispersed in a continuous aqueous phase. The dispersed oil phases can be oil droplets in the aqueous phase.

The O/W architecture or structure, which consists of oil phases dispersed in an aqueous phase, has an external aqueous phase, and therefore if the composition according to the present invention has the O/W architecture or structure, it can provide a pleasant feeling during use because of the feeling of immediate freshness that the aqueous phase can provide.

It is even more preferable that the particle size of the (a) oil be 35 nm or less, preferably 30 nm or less, and more preferably 25 nm or less. The particle size can be measured by a dynamic light scattering method. The particle size measurement can be performed by, for example, the Particle Size Analyzer ELSZ-2000 series, marketed by Otsuka Electronics Co., Ltd.

The particle size can be a volume-average particle diameter or a number-average particle diameter, preferably a volume-average particle diameter.

The composition according to the present invention can be transparent.

The transparency may be measured by measuring the turbidity (for example, turbidity can be measured with a 2100Q (marketed by Hach Company) having a round cell (25 mm in diameter and 60 mm height) and a tungsten filament lamp which can emit visible light (between 400 and 800 nm, preferably from 400 to 500 nm). The measurement can be performed on the undiluted composition. The blank may be determined with distilled water.

The composition according to the present invention has a turbidity of 150 NTU or less, preferably 130 NTU or less and more preferably 110 NTU or less.

### [Process and Use]

It is preferable that the composition according to the present invention be a cosmetic or dermatological composition, preferably a cosmetic composition, and more preferably a cosmetic composition for a keratin substance such as skin.

The composition according to the present invention can be used for a non-therapeutic process, such as a cosmetic process, for treating a keratin substance such as skin, hair, mucous membranes, nails, eyelashes, eyebrows and/or scalp, by being applied to the keratin substance.

Thus, the present invention also relates to a cosmetic process for treating a keratin substance, comprising the step of applying the composition according to the present invention to the keratin substance.

The present invention may also relate to a use of the composition according to the present invention as a cosmetic product or in a cosmetic product such as care products, washing products, make-up products, make-up-removing products, for body and/or facial skin and/or mucous membranes and/or the scalp and/or the hair and/or the nails and/or the eyelashes and/or the eyebrows.

In other words, the composition according to the present invention can be used, as it is, as a cosmetic product. Alternatively, the composition according to the present invention can be used as an element of a cosmetic product. For example the composition according to the present invention can be added to or combined with any other elements to form a cosmetic product.

The care product may be a lotion, a cream, a hair tonic, a hair conditioner, a sun screening agent, and the like. The washing product may be a shampoo, a face wash, a hand wash and the like. The make-up product may be a foundation, a mascara, a lipstick, a lip gloss, a blusher, an eye shadow, a nail varnish, and the like. The make-up-removing product may be a make-up cleansing agent and the like.

Another aspect of the present invention relates to a use of
(b) at least one first polyglyceryl fatty acid ester having an HLB value of 13.0 or more; preferably 13.5 or more, and more preferably 14.0 or more; and
(c) at least one second polyglyceryl fatty acid ester having an HLB value of 10.0 or less, for the manufacture of a composition with a turbidity of 150 NTU or less, preferably 130 NTU or less, and more preferably 110 NTU or less comprising
(a) at least one oil, and
(d) water.

Another aspect of the present invention also relates to a process for preparing a composition with a turbidity of 150 NTU or less, preferably 130 NTU or less, and more preferably 110 NTU or less comprising a step of mixing:
(a) at least one oil;
(b) at least one first polyglyceryl fatty acid ester having an HLB value of 13.0 or more; preferably 13.5 or more, and more preferably 14.0 or more;
(c) at least one second polyglyceryl fatty acid ester having an HLB value of 10.0 or less; and
(d) water.

It is preferable that the mixing step be performed by a so-called low energy process without a special mechanical stirrer, such as a homogenizer which uses a large amount of energy. The low energy process can be performed by simply gently stirring the ingredients (a) to (d).

It is preferable that the above composition in the use and process according to the present invention be in the form of an O/W nano- or micro-emulsion.

The present invention also relates to a use of:
(b) at least one first polyglyceryl fatty acid ester having an HLB value of 13.0 or more, preferably 13.5 or more, and more preferably 14.0 or more; and
(c) at least one second polyglyceryl fatty acid ester having an HLB value of 10.0 or less, in an O/W emulsion comprising
(a) at least one oil, and
(e) water,

in order to make oil droplets of the (a) oil have a particle size of 35 nm or less, preferably 30 nm or less, and more preferably 25 nm or less.

The present invention also relates to a process for making oil droplets of (a) oil in an OAV nano- or micro-emulsion comprising the (a) oil and (d) water have a particle size of 35 nm or less, preferably 30 nm or less, and more preferably 25 nm or less, comprising a step of adding to the emulsion or using in the emulsion:
(b) at least one first polyglyceryl fatty acid ester having an HLB value of 13.0 or more, preferably 13.5 or more, and more preferably 14.0 or more; and
(c) at least one second polyglyceryl fatty acid ester having an HLB value of 10.0 or less.

The above explanations regarding the ingredients (a) to (d), as well as the optional ingredients, for the composition according to the present invention can apply to those for the uses and processes according to the present invention. The explanations regarding the preparation and forms of the composition according to the present invention can also apply to those of the composition recited in the above uses and processes.

### EXAMPLES

The present invention will be described in more detail by way of examples which however should not be construed as limiting the scope of the present invention, which is defined in the appended claims.

### [Examples 1 and 2 and Comparative Example 1]

The following compositions according to Examples 1 and 2 and Comparative Example 1, shown in Table 1, were prepared by mixing the components shown in Table 1 as follows.

### Examples 1 and 2

(1) The ingredients of Phase A were heated at about 60°C and mixed to form a uniform mixture of Phase A ingredients.
(2) The ingredients of Phase B were heated at about 60°C and mixed to form a uniform mixture of Phase B ingredients.
(3) The mixture of the Phase A ingredients and the mixture of the Phase B ingredients were cooled to about 30°C or less.
(4) The mixture of the Phase A ingredients was added to the mixture of the Phase B ingredients under gentle mixing.
(5) The ingredient of Phase C was then added to the mixture obtained in the above step (4) at room temperature to prepare a composition.

### Comparative Example 1

(1) The ingredients of Phase A were heated at about 70°C and mixed to form a uniform mixture of Phase A ingredients.
(2) The ingredients of Phase B were heated at about 70°C and mixed to form a uniform mixture of Phase B ingredients.
(3) The mixture of the Phase A ingredients and the mixture of the Phase B ingredients were cooled to about 30°C or less.
(4) The mixture of the Phase B ingredients was added to the mixture of the Phase A ingredients under gentle mixing.
(5) The ingredients of Phase C were then added to the mixture obtained in the above step (4) at room temperature to prepare a composition.

The compositions according to Examples 1 and 2 and Comparative Example 1 were in the form of an O/W emulsion.

The numerical values for the amounts of the components shown in Table 1 are all based on "% by weight" as active raw materials.

**Table 1**

| | Ingredients | Ex. 1 | Ex. 2 | Comp. Ex. 1 |
|---|---|---|---|---|
| A | PG-4 Caprate (HLB: 14) | 1.00 | 1.03 | - |
| | PG-2 Oleate (HLB: 8) | 0.33 | - | - |
| | PG-2 Isostearate (HLB: 8) | - | 0.30 | - |
| | PG-5 Laurate (HLB: 11) | - | - | 1.33 |
| | Isopropyl Myristate | 1.00 | 1.00 | 1.00 |
| | Phenoxyethanol | - | - | 0.50 |
| | Glycerin | 0.50 | 0.83 | - |
| | Water | 0.50 | 0.17 | - |
| B | Sodium Lauroyl Glutamate | 0.20 | 0.20 | 0.20 |
| | Glycerin | 9.50 | 9.50 | - |
| | Propylene Glycol | 3.00 | 3.00 | 3.00 |
| | Phenoxyethanol | 0.50 | 0.50 | - |
| | Disodium EDTA | 0.10 | 0.10 | 0.10 |
| | Water | 83.17 | 83.17 | 83.67 |
| C | Xanthan Gum | 0.20 | 0.20 | 0.20 |
| | Glycerin | - | - | 10 |
| Total (%) | | 100 | 100 | 100 |
| Stickiness (g) | | -106 | -147 | -217 |
| Turbidity (NTU) | | 32.7 | 102 | 46.7 |
| Average Droplet Size (nm) | | 20.2 | 13.7 | 36.7 |

### [Evaluations]

### (Stickiness)

The stickiness of the compositions according to Examples 1 and 2 and Comparative Example 1 was measured as follows.

### Sample Preparation

30 mg each of the compositions according to Examples 1 and 2 and Comparative Example 1 was evenly applied onto a polyurethane substrate with a size of 3 cm × 3 cm (width) × 4 mm (thickness) (P002-001 from Beaulax Corp).

The composition was dried at 20°C for 30 minutes, and spread with the fingers.

### Measurement

The stickiness of each composition was measured by using Texture Analyzer TA.XT plus with a 20-mm cylinder made of aluminum (P/20). The measurement conditions were as follows.
Test mode: Compression
Pre-Test-Speed: 10 mm/sec
Test Speed: 0.1 mm/sec
Post-Test-Speed: 10 mm/sec
Count: 10

### Data Analysis

The average of 10 counts of negative peaks (g) was determined as the stickiness.

The results are shown in Table 1 as "g". The smaller the g value is, the less sticky the composition is.

### (Turbidity)

The turbidity of the compositions according to Examples 1 and 2 and Comparative Example 1 was measured at room temperature by using a turbidimeter (2100Q portable, Hach Company).

The results are shown in Table 1 as "NTU". The smaller the NTU value is, the more transparent the composition is.

### (Particle Size)

The particle size (nm) of the oil droplets in the compositions according to Examples 1 and 2 and Comparative Example 1 was measured by the Particle Size Analyzer ELSZ-2000ZS (Otsuka Electronics Co., Ltd.).

The results are shown in Table 1.

Examples 1 and 2 show that the use of a combination of the two different types of polyglyceryl fatty acid esters can provide less stickiness.

Comparative Example 1 shows that the use of a single polyglyceryl fatty acid ester provides more stickiness as compared to Examples 1 and 2.

## Claims

1. A composition in the form of a nano- or micro-emulsion, comprising:
(a) at least one oil;
(b) at least one first polyglyceryl fatty acid ester having an HLB value of 13.0 or more, preferably 13.5 or more, and more preferably 14.0 or more;
(c) at least one second polyglyceryl fatty acid ester having an HLB value of 10.0 or less, preferably 9.0 or less, and more preferably 8.0 or less; and
(d) water,
wherein
the composition has a turbidity of 150 NTU or less, preferably 130 NTU or less, and more preferably 110 NTU or less.

2. The composition according to Claim 1, wherein the average HLB of the (b) first polyglyceryl fatty acid ester(s) and the (c) second polyglyceryl fatty acid ester(s) in the composition ranges from 11.5 to 14.0, preferably from 12.0 to 13.5, and more preferably from 12.5 to 13.0.

3. The composition according to Claim 1 or 2, wherein the (a) oil is selected from polar oils.

4. The composition according to any one of Claims 1 to 3, wherein the amount of the (a) oil(s) in the composition ranges from 0.01% to 20% by weight, preferably from 0.05% to 15% by weight, and more preferably from 0.1 % to 10% by weight, relative to the total weight of the composition.

5. The composition according to any one of Claims 1 to 4, wherein the (b) first polyglyceryl fatty acid ester comprises 2 to 4 glycerol units, preferably 3 or 4 glycerol units, and more preferably 4 glycerol units.

6. The composition according to any one of Claims 1 to 5, wherein the fatty acid moiety of the (b) first polyglyceryl fatty acid ester comprises 12 or fewer carbon atoms, preferably 11 or fewer carbon atoms, and more preferably 10 or fewer carbon atoms.

7. The composition according to any one of Claims 1 to 6, wherein the weight ratio of the amount of the (b) first polyglyceryl fatty acid ester(s)/the amount of the (a) oil(s) in the composition ranges from 0.6 to 1.3, preferably from 0.7 to 1.2, and more preferably from 0.8 to 1.1.

8. The composition according to any one of Claims 1 to 7, wherein the (c) second polyglyceryl fatty acid ester comprises 2 to 4 glycerol units, preferably 2 or 3 glycerol units, and more preferably 2 glycerol units.

9. The composition according to any one of Claims 1 to 8, wherein the fatty acid moiety of the (c) second polyglyceryl fatty acid ester comprises 14 or more carbon atoms, preferably 16 or more carbon atoms, and more preferably 18 or more carbon atoms.

10. The composition according to any one of Claims 1 to 9, wherein the weight ratio of the amount of the (c) second polyglyceryl fatty acid ester(s)/the amount of the (a) oil(s) in the composition ranges from 0.1 to 0.9, preferably from 0.2 to 0.7, and more preferably from 0.3 to 0.5.

11. The composition according to any one of Claims 1 to 10, wherein the weight ratio of (the total amounts of the (b) first polyglyceryl fatty acid ester(s) and the (c) second polyglyceryl fatty acid ester(s))/the amount of the (a) oil(s) is 1.6 or less, preferably 1.5 or less, and more preferably 1.4 or less.

12. The composition according to any one of Claims 1 to 11, wherein the composition is free from a polyglyceryl fatty acid ester comprising 5 or more glycerol units.

13. The composition according to any one of Claims 1 to 12, wherein the composition is in the form of an O/W emulsion.

14. The composition according to Claim 13, wherein the particle size of the (a) oil is 35 nm or less, preferably 30 nm or less, and more preferably 25 nm or less.

15. A cosmetic process for treating a keratin substance, comprising the step of applying the composition according to any one of Claims 1 to 14 to the keratin substance.

## Patentansprüche

1. Zusammensetzung in Form einer Nano- oder Mikro-Emulsion, umfassend:
(a) mindestens ein Öl;
(b) mindestens einen ersten Polyglycerylfettsäureester mit einem HLB-Wert von 13,0 oder mehr, vorzugsweise 13,5 oder mehr, und mehr bevorzugt 14,0 oder mehr;
(c) mindestens einen zweiten Polyglycerylfettsäureester mit einem HLB-Wert von 10,0 oder weniger, vorzugsweise 9,0 oder weniger, und mehr bevorzugt 8,0 oder weniger; und
(d) Wasser, wobei
die Zusammensetzung eine Trübung von 150 NTU oder weniger, vorzugsweise 130 NTU oder weniger und mehr bevorzugt 110 NTU oder weniger hat.

2. Zusammensetzung nach Anspruch 1, wobei der durchschnittliche HLB-Wert des/der (b) ersten Polyglycerylfettsäureester(s) und des/der (c) zweiten Polyglycerylfettsäureester(s) in der Zusammensetzung im Bereich von 11,5 bis 14,0, vorzugsweise von 12,0 bis 13,5 und mehr bevorzugt von 12,5 bis 13,0 liegt.

3. Zusammensetzung nach Anspruch 1 oder 2, wobei das (a) Öl aus polaren Ölen ausgewählt ist.

4. Zusammensetzung nach einem der Ansprüche 1 bis 3, wobei die Menge des/der (a) Öls/Öle in der Zusammensetzung im Bereich von 0,01 Gew.-% bis 20 Gew.-%, vorzugsweise von 0,05 Gew.-% bis 15 Gew.-% und mehr bevorzugt von 0,1 Gew.-% bis 10 Gew.-% liegt, bezogen auf das Gesamtgewicht der Zusammensetzung.

5. Zusammensetzung nach einem der Ansprüche 1 bis 4, wobei der (b) erste Polyglycerylfettsäureester 2 bis 4 Glycerineinheiten, vorzugsweise 3 oder 4 Glycerineinheiten und mehr bevorzugt 4 Glycerineinheiten umfasst.

6. Zusammensetzung nach einem der Ansprüche 1 bis 5, wobei der Fettsäureanteil des (b) ersten Polyglycerylfettsäureesters 12 oder weniger Kohlenstoffatome, vorzugsweise 11 oder weniger Kohlenstoffatome und mehr bevorzugt 10 oder weniger Kohlenstoffatome umfasst.

7. Zusammensetzung nach einem der Ansprüche 1 bis 6, wobei das Gewichtsverhältnis der Menge des/der (b) ersten Polyglycerylfettsäureester(s) zur Menge des/der (a) Öls/Öle in der Zusammensetzung im Bereich von 0,6 bis 1,3, vorzugsweise von 0,7 bis 1,2 und mehr bevorzugt von 0,8 bis 1,1 liegt.

8. Zusammensetzung nach einem der Ansprüche 1 bis 7, wobei der (c) zweite Polyglycerylfettsäureester 2 bis 4 Glycerineinheiten, vorzugsweise 2 oder 3 Glycerineinheiten und mehr bevorzugt 2 Glycerineinheiten umfasst.

9. Zusammensetzung nach einem der Ansprüche 1 bis 8, wobei der Fettsäureanteil des (c) zweiten Polyglycerylfettsäureesters 14 oder mehr Kohlenstoffatome, vorzugsweise 16 oder mehr Kohlenstoffatome und mehr bevorzugt 18 oder mehr Kohlenstoffatome umfasst.

10. Zusammensetzung nach einem der Ansprüche 1 bis 9, wobei das Gewichtsverhältnis der Menge des/der (c) zweiten Polyglycerylfettsäureester(s) zur Menge des/der (a) Öls/Öle in der Zusammensetzung im Bereich von 0,1 bis 0,9, vorzugsweise von 0,2 bis 0,7 und mehr bevorzugt von 0,3 bis 0,5 liegt.

11. Zusammensetzung nach einem der Ansprüche 1 bis 10, wobei das Gewichtsverhältnis der Gesamtmengen des/der (b) ersten Polyglycerylfettsäurester(s) und des/der (c) zweiten Polyglycerylfettsäureester(s) zur Menge des/der (a) Öls/Öle 1,6 oder weniger, vorzugsweise 1,5 oder weniger und mehr bevorzugt 1,4 oder weniger beträgt.

12. Zusammensetzung nach einem der Ansprüche 1 bis 11, wobei die Zusammensetzung frei von einem Polyglycerylfettsäureester mit 5 oder mehr Glycerineinheiten ist.

13. Zusammensetzung nach einem der Ansprüche 1 bis 12, wobei die Zusammensetzung in Form einer Ö/W-Emulsion vorliegt.

14. Zusammensetzung nach Anspruch 13, wobei die Teilchengröße des (a) Öls 35 nm oder weniger, vorzugsweise 30 nm oder weniger, und mehr bevorzugt 25 nm oder weniger beträgt.

15. Kosmetisches Verfahren zur Behandlung einer Keratinsubstanz, mit dem Schritt des Auftragens der Zusammensetzung nach einem der Ansprüche 1 bis 14 auf die Keratinsubstanz.

## Revendications

1. Composition sous la forme d'une nano- ou micro-émulsion, comprenant :
(a) au moins une huile ;
(b) au moins un premier ester d'acide gras de polyglycéryle présentant une valeur HLB de 13,0 ou plus, de préférence de 13,5 ou plus, et de manière davantage préférée de 14,0 ou plus ;
(c) au moins un deuxième ester d'acide gras de polyglycéryle présentant une valeur HLB de 10,0 ou moins, de préférence de 9,0 ou moins, et de manière davantage préférée de 8,0 ou moins ; et
(d) de l'eau,
dans laquelle
la composition présente une turbidité de 150 NTU ou moins, de préférence de 130 NTU ou moins, et de manière davantage préférée de 110 NTU ou moins.

2. Composition selon la revendication 1, dans laquelle le HLB moyen du(des) (b) premier(s) ester(s) d'acide gras de polyglycéryle et du(des) (c) deuxième(s) ester(s) d'acide gras de polyglycéryle dans la composition va de 11,5 à 14,0, de préférence de 12,0 à 13,5, et de manière davantage préférée de 12,5 à 13,0.

3. Composition selon la revendication 1 ou 2, dans laquelle la (a) huile est sélectionnée parmi les huiles polaires.

4. Composition selon l'une quelconque des revendications 1 à 3, dans laquelle la quantité de (a) huile (s) dans la composition va de 0,01 % à 20 % en poids, de préférence de 0,05 % à 15 % en poids, et de manière davantage préférée de 0,1 % à 10 % en poids, par rapport au poids total de la composition.

5. Composition selon l'une quelconque des revendications 1 à 4, dans laquelle le (b) premier ester d'acide gras de polyglycéryle comprend 2 à 4 motifs glycérol, de préférence 3 ou 4 motifs glycérol, et de manière davantage préférée 4 motifs glycérol.

6. Composition selon l'une quelconque des revendications 1 à 5, dans laquelle le fragment acide gras du (b) premier ester d'acide gras de polyglycéryle comprend 12 atomes de carbone ou moins, de préférence 11 atomes de carbone ou moins, et de manière davantage préférée 10 atomes de carbone ou moins.

7. Composition selon l'une quelconque des revendications 1 à 6, dans laquelle le rapport pondéral quantité du(des) (b) premier(s) ester(s) d'acide gras de polyglycéryle/quantité de (a) huiles dans la composition va de 0,6 à 1,3, de préférence de 0,7 à 1,2, et de manière davantage préférée de 0,8 à 1,1.

8. Composition selon l'une quelconque des revendications 1 à 7, dans laquelle le (c) deuxième ester d'acide gras de polyglycéryle comprend 2 à 4 motifs glycérol, de préférence 2 ou 3 motifs glycérol, et de manière davantage préférée 2 motifs glycérol.

9. Composition selon l'une quelconque des revendications 1 à 8, dans laquelle le fragment acide gras du (c) deuxième ester d'acide gras de polyglycéryle comprend 14 atomes de carbone ou plus, de préférence 16 atomes de carbone ou plus, et de manière davantage préférée 18 atomes de carbone ou plus.

10. Composition selon l'une quelconque des revendications 1 à 9, dans laquelle le rapport pondéral quantité du(des) (c) deuxième(s) ester(s) d'acide gras de polyglycéryle/quantité de (a) huiles dans la composition va de 0,1 à 0,9, de préférence de 0,2 à 0,7, et de manière davantage préférée de 0,3 à 0,5.

11. Composition selon l'une quelconque des revendications 1 à 10, dans laquelle le rapport pondéral (quantités totales du(des) (b) premier(s) ester(s) d'acide gras de polyglycéryle et du(des) (c) deuxième(s) ester(s) d'acide gras de polyglycéryle)/quantité de (a) huile (s) est de 1,6 ou moins, de préférence de 1,5 ou moins, et de manière davantage préférée de 1,4 ou moins.

12. Composition selon l'une quelconque des revendications 1 à 11, dans laquelle la composition est exempte d'un ester d'acide gras de polyglycéryle comprenant 5 motifs glycérol ou plus.

13. Composition selon l'une quelconque des revendications 1 à 12, dans laquelle la composition est sous la forme d'une émulsion H/E.

14. Composition selon la revendication 13, dans laquelle la taille de particule de (a) l'huile est de 35 nm ou moins, de préférence de 30 nm ou moins, et de manière davantage préférée de 25 nm ou moins.

15. Procédé cosmétique de traitement d'une substance kératineuse, comprenant l'étape d'application de la composition selon l'une quelconque des revendications 1 à 14 sur la substance kératineuse.
